# EUROPEAN PATENT APPLICATION

(11) **EP 4 707 286 A1**
(43) Date of publication of application: **11.03.2026**
(21) Application number: 24800127.3
(22) Date of filing: 01.05.2024
(51) Int. Cl.: C07H 19/10

(54) **HIGHLY LIPID-SOLUBLE NUCLEOTIDE BUILDING BLOCK FOR OLIGONUCLEOTIDE SYNTHESIS, AND SYNTHESIS OF OLIGONUCLEOTIDES USING SAME**

(30) Priority: 01.05.2023 US 202363499274 P
(71) Applicant: Natias Inc., Kobe-shi, Hyogo 650-0047 (JP)
(72) Inventor: KATAOKA, Masanori, Kobe-shi, Hyogo 650-0047 (JP); TSUJI, Yohei, Kobe-shi, Hyogo 650-0047 (JP); FUKUI, Chiharu, Kobe-shi, Hyogo 650-0047 (JP); YAMASHITA, Rio, Kobe-shi, Hyogo 650-0047 (JP); MATSUMOTO, Keiichi, Kobe-shi, Hyogo 650-0047 (JP); WATARI, Mizuki, Kobe-shi, Hyogo 650-0047 (JP); AKAI, Yuto, Higashiosaka-shi, Osaka 577-0056 (JP); KODAMA, Hidehiko, Higashiosaka-shi, Osaka 577-0056 (JP)
(74) Representative: Elzaburu S.L.P.
(86) International application number: PCT/JP2024/016835
(87) International publication number: WO 2024/228396

(57) **Abstract**

The present invention provides a highly lipophilic nucleotide building block for oligonucleotide synthesis having a structure represented by the following Formula (III) or Formula (VI). In the Formula (III) and Formula (VI), R¹ is at least one of Tr, MMTr, DMTr, TMTr, TBDPS, benzoyl, isobutyryl, dmf, and Ac, provided that, in the Formula (III) and Formula (VI), when both OR² and Y are one of OMMTr, ODMTr, and OTMTr, R¹ is optionally free from a protecting group; and R² is one of Tr, MMTr, DMTr, TMTr, TBDPS, Fmoc, and 1-pyrenylmethyl.

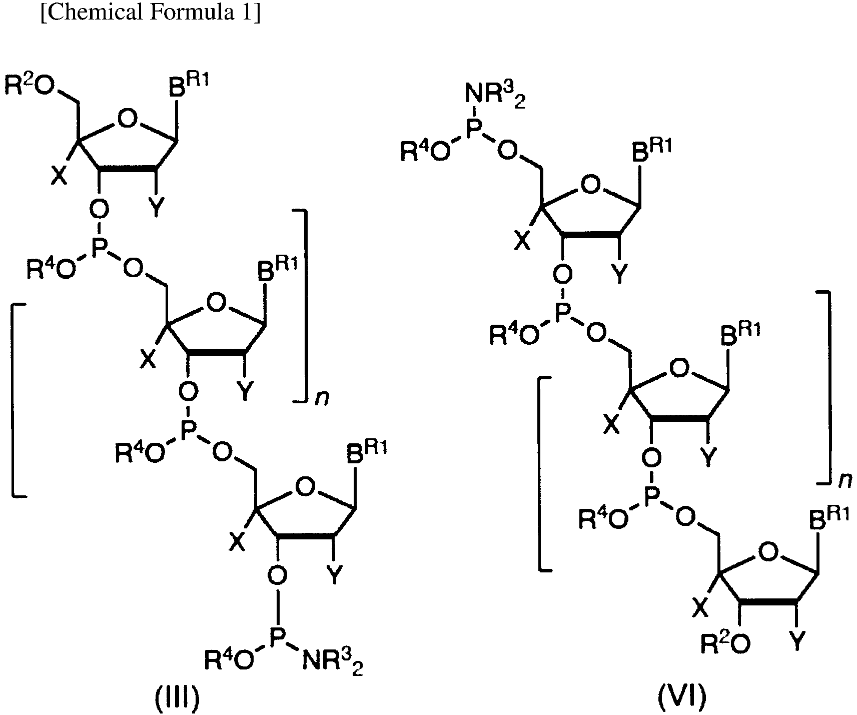

## Description

### [Technical Field]

The present invention relates to a highly lipophilic nucleotide building block for oligonucleotide synthesis and to the synthesis of oligonucleotides using the same.

### [Background Art]

In recent years, increasing attention has been paid to nucleic acid drugs based on naturally occurring or modified oligonucleotides as a basic backbone. In order to obtain a nucleic acid drug designed to exert a desired effect, chemical synthesis methods have been widely employed.

As one chemical synthesis method, a liquid-phase synthesis method is known, in which all reactions proceed in a liquid phase and which is used for the synthesis of a relatively short-chain oligonucleotide. Recently, attempts have been made to apply a fluorous tag, which is a tag with high hydrophobicity (a substituent with affinity for fluorocarbons) to this liquid-phase synthesis method (PTL 1 and PTL 2).

### [Citation List]

### [Patent Literature]

[PTL 1] PCT International Publication No. WO 2005/070859
[PTL 2] PCT International Publication No. WO 2017/086397

### [Summary of Invention]

### [Technical Problem]

Furthermore, in order to manufacture a nucleic acid active pharmaceutical ingredient (API) at the Good Manufacturing Practice (GMP) grade and to stably supply it as a pharmaceutical product, it is necessary to be able to synthesize oligonucleotides in a kilogram scale and with high purity. However, a purification step by column chromatography or the like is complicated in many cases, and therefore, it is not easy to synthesize oligonucleotides in large amounts, particularly long-chain oligonucleotides exceeding 50-mers.

For synthesizing oligonucleotides on a large scale, a liquid-phase synthesis method that allows the use of a commonly available reactor is advantageous. Even in a liquid-phase synthesis method using such a reactor, for example, in the synthesis of oligonucleotides in an amount exceeding 100 g per batch, many steps are required before the desired product is obtained.

In detail, the liquid-phase batch synthesis method includes a step of chain elongation, a step of deprotection, a step of removal of excess reagents and by-products generated in the reaction, a step of purification, a step of desalting, and the like. In the case of the synthesis on a large scale, reaction apparatuses, purification apparatuses, and other equipment corresponding to each step and its scale are required. As the multi-step synthesis proceeds, impurities such as unreacted reagents and reaction by-products are generated, and the removal of such impurities requires purification by column chromatography or the like. Accordingly, in order to manufacture oligonucleotides in a large amount, process improvement and cost reduction are demanded.

In addition, the fluorous tags used in PTL 1 and PTL 2 have a complex structure and are not readily available, which increases the costs.

In view of such circumstances, the present invention has been made to provide a highly lipophilic nucleotide building block for oligonucleotide synthesis and a method of synthesizing a protected oligonucleotide using the same, which enable the synthesis of oligonucleotides on a large scale and purification in a more convenient manner.

### [Solution to Problem]

In order to solve the above problems, the highly lipophilic nucleotide building block for oligonucleotide synthesis and the method of synthesizing oligonucleotides using the same according to the present invention employ the following means.

A first aspect of the present invention provides a highly lipophilic monomer building block for oligonucleotide synthesis including a structure represented by the following Formula (I) or Formula (II).

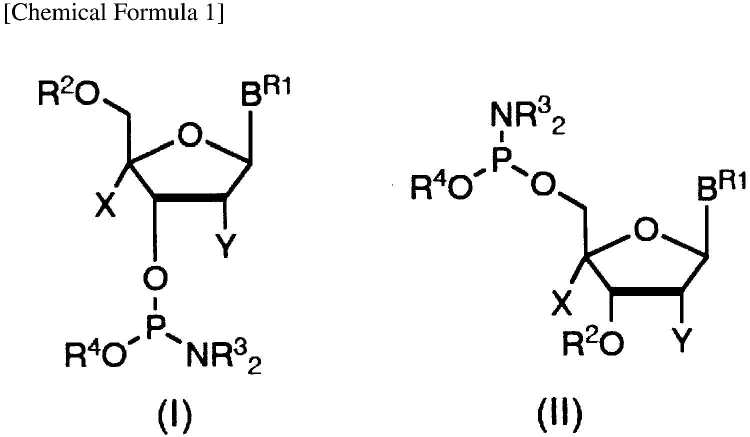

In the Formula (I) and Formula (II),
B is a naturally occurring or non-naturally occurring base;
R¹ is at least one of Tr, MMTr, DMTr, TMTr, TBDPS, TBS, Fmoc, benzoyl, isobutyryl, dmf, and Ac, provided that, in the Formula (II), when both OR² and Y are one of OMMTr, ODMTr, and OTMTr, R¹ is optionally free from a protecting group;
R² is one of Tr, MMTr, DMTr, TMTr, TBDPS, TBS, Fmoc, and 1-pyrenylmethyl;
R³ is CH₃, CH₂CH₃, or CH(CH₃)₂, or NR³₂ forms a ring that is -N(CH₂CH₂)₂ or - N(CH₂CH₂)₂O;
R⁴ is independently one of CH₂CH₂CN, CH₂CH=CH₂, CH₂(CF₂)₆CF₃, and CH₂(CF₂)₄CF₃;
X is H, or forms an X-Y bond together with Y; and
Y is one of F, OCH₃, OMOE, OTBDMS, and OR², or forms an X-Y bond, wherein X-Y is CH₂O, CH₂CH₂O, CH₂NH, CH₂NR', or CH₂N-N=NH₂, and R' is independently H, alkyl, carbamate, amide group, or substituted silyl.

In the first aspect described above, B^{R} may be independently selected from A^{DMTr}, A^{TMTr}, A^{Ac}, C^{DMTr}, C^{TMTr}, C^{Ac}, G^{MMTr}, G^{Tr}, G^{ibu/TBDPS}, G^{dmf}, U^{Tr}, U^{TBDPS}, T^{MMTr}, and T^{TBDPS}.

A second aspect of the present invention provides a highly lipophilic nucleotide building block for oligonucleotide synthesis including a structure represented by the following Formula (III) or Formula (VI):

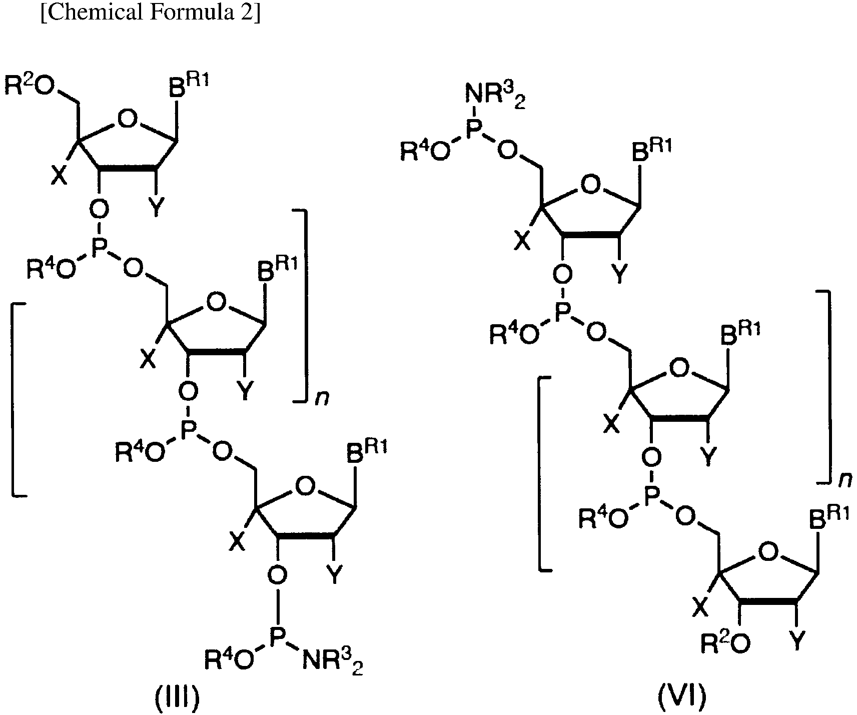

In the Formula (III) and Formula (VI),
B is a naturally occurring or non-naturally occurring base;
R¹ is at least one of Tr, MMTr, DMTr, TMTr, TBDPS, TBS, Fmoc, benzoyl, isobutyryl, dmf, and Ac, provided that, in the Formula (III) and Formula (VI), when both OR² and Y are one of OMMTr, ODMTr, and OTMTr, R¹ is optionally free from a protecting group;
R² is one of Tr, MMTr, DMTr, TMTr, TBDPS, TBS, Fmoc, and 1-pyrenylmethyl;
R³ is CH₃, CH₂CH₃, or CH(CH₃)₂, or NR³₂ forms a ring that is -N(CH₂CH₂)₂ or - N(CH₂CH₂)₂O;
R⁴ is independently one of CH₂CH₂CN, CH₂CH=CH₂, CH₂(CF₂)₆CF₃, and CH₂(CF₂)₄CF₃;
X is H, or forms an X-Y bond together with Y;
Y is one of F, OCH₃, OMOE, OTBDMS, and OR², or forms an X-Y bond, wherein X-Y is CH₂O, CH₂CH₂O, CH₂NH, CH₂NR', or CH₂N-N=NH₂, and R' is independently H, alkyl, carbamate, amide group, or substituted silyl; and
n is an integer from 0 to 46.

In the second aspect described above, B^{R} may be independently selected from A^{DMTr}, A^{TMTr}, A^{Ac}, A, C^{DMTr}, C^{TMTr}, C^{Ac}, C, G^{DMTr}, G^{Tr}, G^{ibu/TBDPS}, G^{dmf}, G, U^{Tr}, U^{TBDPS}, U, T^{MMTr}, T^{TBDPS}, and T.

In the second aspect described above, R¹ may be protected with MMTr, DMTr, or TMTr.

In the second aspect described above, n may be an integer from 0 to 10.

A third aspect of the present invention provides a method of synthesizing an oligonucleotide using the highly lipophilic nucleotide monomer block according to the first aspect described above or the highly lipophilic nucleotide building block according to the second aspect described above, including:
(a) a step of chain elongation in which an amidite portion of the highly lipophilic nucleotide monomer block represented by the above Formula (I) or Formula (II) or the highly lipophilic nucleotide monomer block represented by the above Formula (III) or Formula (VI) is condensed with a hydroxyl group of a protected nucleoside or a protected nucleotide; and
(b) a step of deprotection of a protecting group of a hydroxyl group at one end of a chain-elongated product obtained in the chain elongation step,
   wherein, the method includes, after performing the chain elongation step and the deprotection step at least once or a predetermined number of times to obtain a protected oligonucleotide of a desired length,
(c) a step of deprotection of a phosphate protecting group, a protecting group of a nucleobase, and a protecting group of a hydroxyl group at a 3'- or 5'-end of the protected oligonucleotide to synthesize a protected oligonucleotide having a highly lipophilic protecting group at at least two positions;
(d) a step of separating the protected oligonucleotide having a highly lipophilic protecting groups at at least two positions; and
(e) a step of deprotecting the separated protected oligonucleotide and purifying the obtained oligonucleotide.

In the third aspect described above, the number of nucleosides contained in the protected oligonucleotide may be in a range of 10 to 150.

In the third aspect described above, the highly lipophilic protecting groups at at least two positions in the protected oligonucleotide obtained after the deprotection step (c) may be Tr, MMTr, DMTr, TMTr, Fmoc, or TBDPS protecting groups.

In the third aspect described above, the chain elongation step (a), the deprotection step (b), and the deprotection step (c) may be carried out in a liquid phase.

### [Advantageous Effects of Invention]

By using the highly lipophilic nucleotide building block according to the present invention, it is possible to synthesize oligonucleotides on a large scale and purify the resulting oligonucleotides in a more convenient manner.

### [Brief Description of Drawings]

[Fig. 1] Fig. 1 is a partially enlarged view of an HPLC chart of an oligonucleotide N-mer and (N-1)-mer synthesized and purified by a conventional method.
[Fig. 2A] Fig. 2 is an HPLC chart illustrating before and after purification of a 21-mer having a sequence shown in the Sequence ID No. 1, and Fig. 2A shows the HPLC chart of a crude product that is 5'-bis-tritylated.
[Fig. 2B] Fig. 2 is the HPLC chart illustrating before and after purification of the 21-mer having the sequence shown in the Sequence ID No. 1, and Fig. 2B shows the HPLC chart after the product following detritylation is purified.
[Fig. 3A] Fig. 3 is an HPLC chart illustrating before and after purification of a 21-mer having a sequence shown in the Sequence ID No. 4, and Fig. 3A shows the HPLC chart of a crude product that is 5'-bis-tritylated.
[Fig. 3B] Fig. 3 is the HPLC chart illustrating before and after purification of the 21-mer having the sequence shown in the Sequence ID No. 4, and Fig. 3B shows the HPLC chart after the product following detritylation is purified.

### [Description of Embodiments]

Hereinafter, one embodiment of the highly lipophilic nucleotide building block according to the present invention and a protected oligonucleotide using the same will be described.

In the present specification, the term "nucleotide building block" refers to a nucleotide unit which includes a dimer or more of nucleotides, has a phosphoramidite structure at the 3'- or 5'-end, and serves as a synthetic block for forming a longer-chain nucleotide by undergoing a condensation reaction with a nucleoside, nucleotide, or the like with the 5'- or 3'-hydroxyl group not protected.

In the present specification, the term "nucleotide monomer block" refers to a nucleotide unit which includes one nucleoside, has a phosphoramidite structure at the 3'- or 5'-end, and serves as a synthetic block for forming a long-chain nucleotide by undergoing a condensation reaction with a nucleoside, nucleotide, or the like with the 5'- or 3'-hydroxyl group not protected.

The highly lipophilic nucleotide monomer block in the present embodiment has a structure represented by the following Formula (I) or Formula (II).

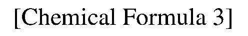

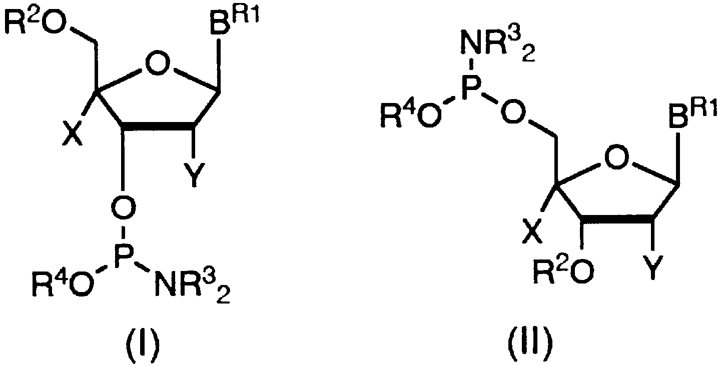

In the above Formula (I) and Formula (II), B is a naturally occurring or non-naturally occurring base. R¹, which is a protecting group for the nucleoside base, is one of Tr, MMTr, DMTr, TMTr, TBDPS, TBS, Fmoc, benzoyl, isobutyryl, dmf, and Ac, provided that, in the Formula (II), when both OR² and Y are one of OMMTr, ODMTr, and OTMTr, R¹ is optionally free from a protecting group. R², which is a protecting group for the 5'- or 3'-hydroxyl group, is one of Tr, MMTr, DMTr, TMTr, TBDPS, TBS, Fmoc, and 1-pyrenylmethyl. R³ of the phosphoramidite portion is CH₃, CH₂CH₃, or CH(CH₃)₂, or NR³₂ forms a ring that is - N(CH₂CH₂)₂ or -N(CH₂CH₂)₂O.

R⁴, which is a protecting group of the phosphate portion, is independently one of CH₂CH₂CN, CH₂CH=CH₂, CH₂(CF₂)₆CF₃, and CH₂(CF₂)₄CF₃. X, which is bonded to a 4'-carbon of a ribose or 2'-deoxyribose ring of the nucleoside, is H or forms an X-Y bond together with Y. Y, which is bonded to a 2'-carbon of a furanose ring of the nucleoside, is one of F, OCH₃, OMOE, OTBDMS, and OR², or forms an X-Y bond, and X-Y is CH₂O, CH₂CH₂O, CH₂NH, CH₂NR', or CH₂N-N=NH₂. Here, R' is independently H, alkyl, carbamate, amide group, or substituted silyl.

In the structure represented by the above Formula (I) and Formula (II), B^{R} may be independently selected from A^{DMTr}, A^{TMTr}, A^{Ac}, C^{DMTr}, C^{TMTr}, C^{Ae}, G^{DMTr}, G^{Tr}, G^{MMTr/TBDPS}, G^{dmf}, U^{Tr}, U^{TBDPS}, U, T^{MMTr}, T^{TBDPS}, and T. In other words, the protecting group for the nucleobase portion of the nucleoside may be selected from Tr, MMTr, DMTr, TMTr, TBDPS, dmf, and Ac.

The Tr, MMTr, DMTr, TMTr, Fmoc, and TBDPS groups themselves are highly lipophilic, and therefore, protected nucleosides having such groups as protecting groups are also highly lipophilic. In the present embodiment, in the structure represented by the Formula (II), that is, in the monomer block having a ribonucleoside as the basic backbone, when both the 2'-hydroxyl group and the 3'-hydroxyl group have one of MMTr, DMTr, and TMTr as a protecting group, the nucleobase portion may have no protecting group, or a protecting group such as dmf or Ac, which has lower lipophilicity as compared to the trityl-type protecting groups described above, may be used. This is because, by having any of MMTr, DMTr, and TMTr as a protecting group at both the 2'-hydroxyl group and the 3'-hydroxyl group, the lipophilicity required for the oligonucleotide synthesis in the present embodiment can be obtained.

Since the MMTr, DMTr, and TMTr protecting groups mentioned above are commonly used protecting groups in nucleotide chemical synthesis, methods for deprotection thereof are known well. Therefore, by appropriately combining the protecting group for the terminal hydroxyl group, the protecting group for the nucleobase portion, and the protecting group for the phosphate portion in the nucleotide monomer block, and by deprotecting them in an appropriate order, it is possible to achieve purification at high purity without increasing the number of steps in the oligonucleotide synthesis. The deprotection steps and the purification steps will be described later.

The highly lipophilic nucleotide building block for oligonucleotide synthesis in the present embodiment has the structure represented by the following Formula (III) or Formula (VI).

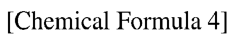

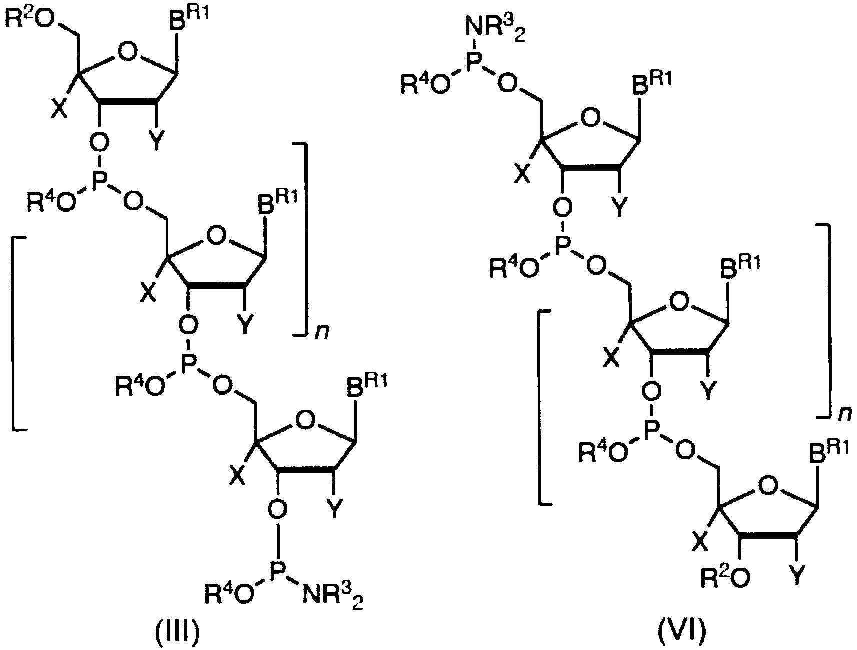

In the Formula (III) and Formula (VI), B is a naturally occurring or non-naturally occurring base. R¹, which is a protecting group for B, is any one of Tr, MMTr, DMTr, TMTr, TBDPS, TBS, Fmoc, dmf, and Ac, or indicates no protecting group.

Here, in the highly lipophilic nucleotide building block according to the present embodiment, in the structure represented by the Formula (III) and Formula (VI), at least two of R¹, R², and R⁴ are any of Tr, MMTr, DMTr, TMTr, Fmoc, and TBDPS. By having these protecting groups in such numbers, it is possible to achieve purification at high purity without increasing the number of steps in the oligonucleotide synthesis using the highly lipophilic nucleotide building block according to the present embodiment. The deprotection steps and the purification steps will be described later.

Further, in the structure represented by the Formula (III) and Formula (VI), when at least two of R¹, R², R⁴, and Y are any of Tr, MMTr, DMTr, TMTr, Fmoc, and TBDPS, R¹ does not necessarily need to have a protecting group.

R², which is a protecting group for the 5'- or 3'-hydroxyl group, is one of Tr, MMTr, DMTr, TMTr, TBDPS, TBS, Fmoc, and 1-pyrenylmethyl. R³ of the phosphoramidite portion is CH₃, CH₂CH₃, or CH(CH₃)₂, or NR³₂ forms a ring that is -N(CH₂CH₂)₂ or -N(CH₂CH₂)₂O.

R⁴, which is a protecting group for the phosphate portion, is independently one of CH₂CH₂CN, CH₂CH=CH₂, CH₂(CF₂)₆CF₃, and CH₂(CF₂)₄CF₃.

X, which is bonded to a 4'-carbon of a ribose or 2'-deoxyribose, is H or forms an X-Y bond together with Y. Y, which is bonded to a 2'-carbon of a furanose ring of the nucleoside, is one of F, OCH₃, OMOE, OTBDMS, and OR², or forms an X-Y bond, and X-Y is CH₂O, CH₂CH₂O, CH₂NH, CH₂NR', or CH₂N-N=NH₂. Here, R' is independently H, alkyl, carbamate, amide group, or substituted silyl.

In the structure represented by the Formula (III) and Formula (VI), n is an integer from 0 to 46, preferably from 0 to 10, more preferably from 0 to 5, and even more preferably from 0 to 3.

As a nucleoside base (also referred to as a nucleobase) in the present embodiment, naturally occurring bases, such as an adenyl group, a guanyl group, a cytosinyl group, a thyminyl group, and an uracil group, as well as modified bases, such as a 5-methylcytosinyl group, a 5-fluorouracil group, a 7-methylguanyl group, and a 7-deazaadenyl group, can be used. The term "non-naturally occurring base" in the present specification includes bases having a reactive functional group, such as an amino group, a carbonyl group, a hydroxyl group, and a thiol group. A highly lipophilic protecting group may be introduced to the reactive functional group described above, if necessary.

The method of synthesizing an oligonucleotide using the highly lipophilic monomer building block or the highly lipophilic nucleotide building block according to the present embodiment firstly performs (a) a step of chain elongation in which an amidite portion of the highly lipophilic nucleotide monomer block represented by the above Formula (I) or Formula (II) or the highly lipophilic nucleotide monomer block represented by the above Formula (III) or Formula (VI) is condensed with a hydroxyl group of a protected nucleoside or a protected nucleotide. Subsequently, the method performs (b) a step of deprotection of a protecting group of a hydroxyl group at one end of a chain-elongated product obtained in the chain elongation step.

In the chain elongation step, the phosphoramidite portion of the highly lipophilic monomer building block or the highly lipophilic nucleotide building block is activated by an activator, and a condensation reaction is carried out with the hydroxyl group of the protected nucleoside or the protected nucleotide. Typical examples of activators include, but are not limited to, 1H-tetrazole derivatives, such as 1H-tetrazole and S-ethylthiotetrazole, imidazole derivatives, such as dicyanoimidazole and dichloroimidazole, and salts of sulfonic acids with azoles or tertiary amines. The reaction is carried out in a dried solvent such as dichloromethane, acetonitrile, tetrahydrofuran, DMF, or toluene.

The chain-elongated product obtained in the chain elongation step may, if necessary, be subjected to oxidation or sulfurization of a phosphite portion, and then provided for the subsequent deprotection step (b).

As described above, the number of nucleosides contained in the highly lipophilic nucleotide building block represented by the structure of Formula (III) and Formula (VI) is from 4 to 48. Using these, by performing a single condensation reaction (also referred to as a chain elongation reaction) between the amidite portion of the building block according to the present embodiment and the unprotected hydroxyl group of the protected nucleoside or the protected nucleotide as the condensation partner, the length of the resulting oligonucleotide chain can be extended by at least four bases, up to 48 bases, at once.

Thus, according to the method of synthesizing an oligonucleotide using the highly lipophilic nucleotide building block according to the present embodiment, by merely repeating (a) the chain elongation step and (b) the deprotection step fewer times than the number of nucleosides to be contained in the desired oligonucleotide, the number of nucleosides contained in the oligonucleotide can be in a range of 10 to 150.

This represents a great advantage as compared with commonly used chemical synthesis methods for oligonucleotides, in which chain elongation reactions must be carried out stepwise, for one base at a time, in that the number of steps can be reduced. Moreover, by reducing the number of steps, it is also possible to reduce an amount of waste liquid generated, thereby decreasing the environmental burden.

The method of synthesizing an oligonucleotide according to the present embodiment, after carrying out the chain elongation step and the deprotection step at least once, or repeating the steps a predetermined number of times, to obtain a protected oligonucleotide of the desired length, performs (c) a step of deprotection of a phosphate protecting group, a protecting group of a nucleobase, and a protecting group of a hydroxyl group at a 3'- or 5'-end of the protected oligonucleotide to synthesize a protected oligonucleotide having a highly lipophilic protecting group at at least two positions; (d) a step of separating the protected oligonucleotide having a highly lipophilic protecting groups at at least two positions; and (e) a step of deprotecting the separated protected oligonucleotide and purifying the obtained oligonucleotide.

In the nucleotide building block or nucleotide monomer block according to the present embodiment, in a case where the backbone is a 2'-deoxyribonucleoside, highly lipophilic protecting groups are present at the 5'-end and two positions in the nucleobase portion, and in a case where the backbone is a ribonucleoside, highly lipophilic protecting groups are present at the 5'-end, the 2'-hydroxyl group, and at least two positions in the nucleobase portion.

In the method of synthesizing an oligonucleotide using the nucleotide building block or the nucleotide monomer block according to the present embodiment, in the step (c), the other protecting groups are removed so that the highly lipophilic protecting groups remain at at least two positions.

In the method of synthesizing an oligonucleotide using the highly lipophilic nucleotide building block according to the present embodiment, the highly lipophilic protecting groups present at at least two positions in the protected oligonucleotide obtained after the deprotection step (c) may be Tr, MMTr, DMTr, TMTr, Fmoc, or TBDPS protecting groups. By having a plurality of such highly lipophilic protecting groups, in performing the subsequent separation step (d), the desired product can be readily separated from by-products and waste reagents by simple purification operations, such as hydrophobic chromatography using C4, C6, or C8 silica gel, reverse-phase chromatography using C18 reverse-phase silica gel or the like, a combination of hydrophobic chromatography by hydrophilic interaction and solid-phase extraction, solid-liquid separation, aqueous layer-organic layer separation, or gel filtration.

By selecting the deprotection conditions in the deprotection step (b), it is possible to synthesize a protected oligonucleotide in which only the highly lipophilic protecting groups described above remain. In other words, the highly lipophilic nucleotide building block according to the present embodiment is designed to contain the number of highly lipophilic protecting groups necessary for enabling easy separation in the separation step (d). Depending on the sequence and the length of the desired oligonucleotide, various building blocks can be combined and used for the synthesis.

By subjecting the protected oligonucleotide obtained in the separation step (d) to the step of deprotection of the remaining protecting groups and purification, the desired oligonucleotide can be obtained. In this step, the deprotection can be carried out under conditions commonly used for the remaining protecting groups. For example, when trityl-type protecting groups, such as MMTr and DMTr, remain, the deprotection is carried out using an aqueous solution of trifluoroacetic acid.

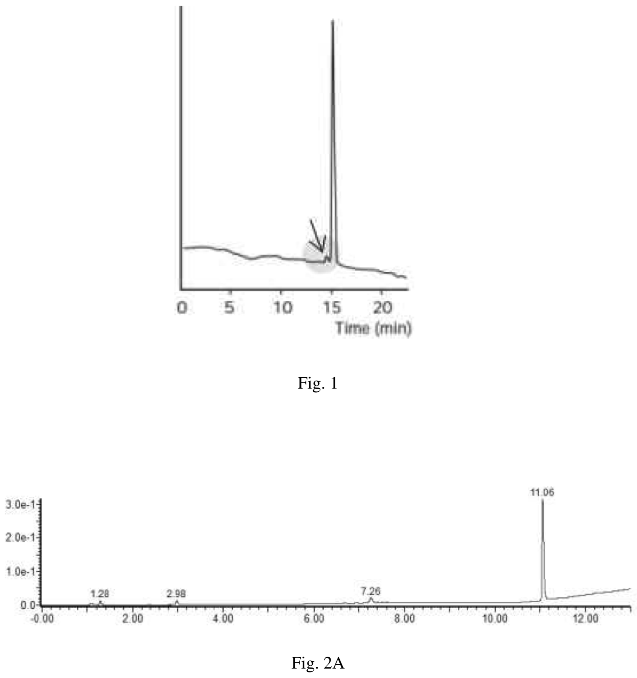

When N-mer oligonucleotides are synthesized using commonly used phosphoramidites, (N-1)-mers are generated as by-products. Since the (N-1)-mers have almost the same physical properties as those of the desired N-mers, as shown in Fig. 1, it is extremely difficult to remove the (N-1)-mers in the separation and purification at the final stage.

In contrast, the highly lipophilic nucleotide monomer block according to the present embodiment has at least two highly lipophilic protecting groups. Therefore, in the synthesis of an oligonucleotide, after proceeding with chain elongation by using, for example, commercially available phosphoramidites, the highly lipophilic nucleotide monomer block is used in the final chain elongation step. In the case of synthesizing an N-mer oligonucleotide, the protected N-mer oligonucleotide containing the highly lipophilic nucleotide monomer at the 5'- or 3'-end according to the present embodiment has higher lipophilicity, that is, higher hydrophobicity, as compared with the (N-1)-mer that remained without undergoing the chain elongation. By utilizing this difference in the hydrophobicity, in the separation, the peak of the N-mer containing the highly lipophilic nucleotide monomer and the peak of the (N-1)-mer to be separated can be more widely separated. Thus, even by a simple separation step, short-chain by-products can be readily removed, and the purity of the final desired product can be further increased.

The advantage that it is possible to utilize the difference in the hydrophobicity between the peak of the desired N-mer and the peak of the by-products to be separated, as described above, can likewise be obtained when using the highly lipophilic nucleotide building block according to the present embodiment. Since the highly lipophilic nucleotide building block contains from two to 46 nucleosides, by adjusting the positions and the number of the plurality of highly lipophilic protecting groups to remain, the separation and the subsequent deprotection treatment can be efficiently carried out based on the length of the desired oligonucleotide.

In the present specification, the terms "highly lipophilic" and "high lipophilicity" refer to properties indicating high affinity for organic solvents (for example, hydrophobic solvents such as aliphatic hydrocarbon-based solvents) and, conversely, low affinity for water. In the present specification, an oligonucleotide in which protecting groups having ten or more carbon atoms are locally introduced at two or more positions is referred to as an oligonucleotide having "highly lipophilic" protecting groups.

In the method of synthesizing an oligonucleotide according to the present embodiment, the chain elongation step (a), the deprotection step (b), and the deprotection step (c) may be carried out in a liquid phase (hereinafter, also referred to as the liquid-phase synthesis method). As compared with the solid-phase synthesis method, in which coupling reactions are carried out on a solid-phase resin, the liquid-phase synthesis method allows synthesis on a large scale (100 g or more). Therefore, oligonucleotides can be synthesized at a lower cost.

Moreover, the chain elongation step (a), the deprotection step (b), and the deprotection step (c) can all be carried out in a liquid phase. Furthermore, as described above, the separation step (d) can also be carried out with simple equipment, such as hydrophobic chromatography using C4, C6, or C8 silica gel, reverse-phase chromatography using C18 reverse-phase silica gel or the like, a combination of hydrophobic chromatography by hydrophilic interaction and solid-phase extraction, solid-liquid separation, aqueous layer-organic layer separation, or gel filtration. Thus, in the method of synthesizing an oligonucleotide using the highly lipophilic nucleotide building block according to the present embodiment, the operations from the chain elongation reaction of the raw material to the separation and purification steps, through the oxidation or sulfurization reactions and the deprotection reactions, can be continuously executed in a closed system by a liquid-phase flow system. In other words, oligonucleotide synthesis can be continuously carried out in a liquid-phase flow system from the raw materials in a single integrated process.

In the method of synthesizing a nucleotide monomer block according to the embodiment of the present invention, the synthesis of the nucleoside having a plurality of highly lipophilic protecting groups is carried out in the same manner as described for the method of synthesizing a nucleotide monomer block. The method of using this as a building block can be carried out in the same manner as in the methods of synthesizing a blockmer described in PCT International Publication Nos. WO 2019/212061 and WO 2020/235658.

In the nucleotide monomer block and the nucleotide building block of the present embodiment, highly lipophilic protecting groups can be introduced by using a commercially available reagent for a protecting group. Furthermore, depending on the purpose, the number of highly lipophilic protecting groups to be introduced can be easily changed. Therefore, in order to obtain the desired lipophilicity, it is possible to obtain the desired product more easily at a lower cost than introduction of a highly lipophilic tag or linker that is not commercially available and thus would require complicated separate steps for synthesis.

In the synthesis of the highly lipophilic nucleotide building block of the present embodiment, the solubility of intermediates in the synthesis of the oligonucleotide can be adjusted by controlling the number of highly lipophilic protecting groups to be introduced. This makes the synthesis method more versatile. Furthermore, depending on the length and the sequence of the monomer or the oligonucleotide to be synthesized, the highly lipophilic protecting groups can be introduced into the nucleoside base portion, or into the protecting groups at the 5'-end, 3'-end, or 2'-hydroxyl group of the nucleoside.

Furthermore, according to the method of synthesizing an oligonucleotide using the highly lipophilic nucleotide building block of the present embodiment, in synthesizing the same N-mer oligonucleotide, the number of required steps can be reduced as compared with the commonly used liquid-phase synthesis methods in which elongation is carried out for one base at a time. Accordingly, the yield of the oligonucleotide of the desired length can be improved.

The method of synthesizing the nucleotide monomer block according to the embodiment of the present invention will be illustratively described. A process was examined in which the hydroxyl groups of thymidine were acetylated with acetic anhydride to synthesize 3',5'-diacetylthymidine, followed by protecting the base portion with MMTr. The acetyl groups were deprotected by hydrolysis using potassium carbonate, and the hydroxyl group at the 5'-position was protected with MMTr using MMTrCl. Each step proceeded with good yield, and it was confirmed that the desired bis-MMTr derivative of thymidine could be synthesized on a scale of 100 g. Details of the reaction conditions will be described in the Examples.

### [Examples]

### [Example 1] Bis-MMTr protection and 3'-amidite formation of thymidine

### 1-1. Diacetylation of thymidine

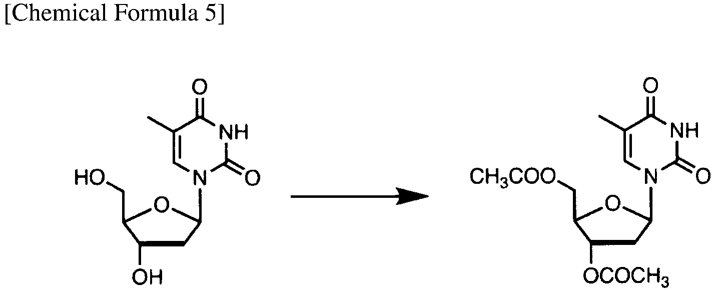

Into a 2 L single-neck round-bottom flask, thymidine (263.4 mmol), pyridine (18 V), and acetic anhydride (7 V) were placed, and the mixture was allowed to react for four hours. After confirming the disappearance of thymidine and the formation of the desired product by TLC (hexane/ethyl acetate = 1/2, the Rf value of thymidine: 0, the Rf value of the desired product: 0.19), the reaction mixture was concentrated to remove the pyridine and the acetic anhydride. The concentrate was diluted with ethyl acetate and was neutralized with an aqueous solution of sodium bicarbonate. The organic layer was washed with water and saturated saline, and was dried over sodium sulfate. After filtration and concentration, the solution was then purified by column chromatography (Biotage Sfar HC Duo, 350 g × 3, hexane/ethyl acetate = 1/1 to 0/1) to obtain 3',5'-diacetylthymidine in 61.7 g (at a yield of 72%).

### 1-2. N3-MMTr Protection of 3',5'-diacetylthymidine

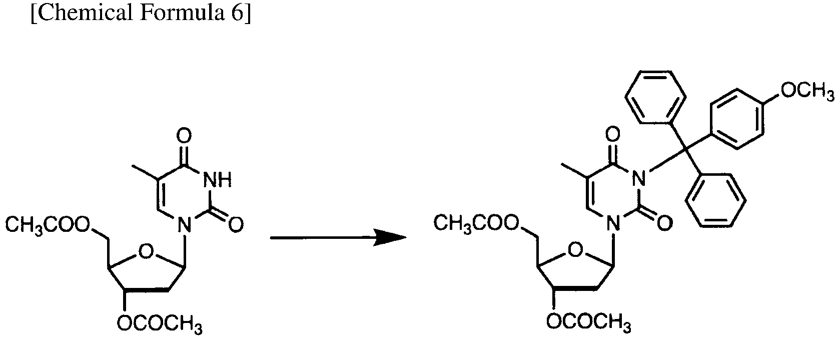

Into a 1 L single-neck round-bottom flask, the 3',5'-diacetylthymidine (96.2 mmol), DBU (1.80 eq.), and pyridine (10 V) were placed. To this solution, MMTrCl (1.60 eq.) was added, and the mixture was stirred at 45°C for 20 hours. After confirming the formation of the desired product by TLC (hexane/ethyl acetate = 1/1, the Rf value of the raw ingredient: 0.08, the Rf value of the desired product: 0.33), the mixture was concentrated to remove the pyridine. The concentrate was diluted with ethyl acetate, washed with an aqueous solution of sodium bicarbonate, water, and saturated saline, and dried over sodium sulfate. After filtration and concentration, the solution was then purified by column chromatography (Biotage Sfar HC Duo, 350 g × 2, hexane/ethyl acetate = 4/1 to 1/1) to obtain N3-MMTr-3',5'-diacetylthymidine in 50.6 g (at a yield of 88%).

### 1-3. Deacetylation of 3-MMTr-3',5'-diacetylthymidine

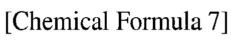

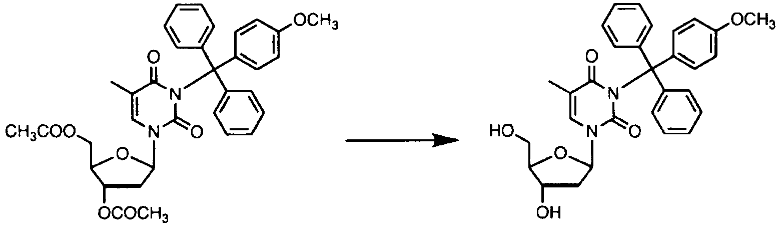

Into a 1 L single-neck round-bottom flask, the 3-MMTr-3',5'-diacetylthymidine (85.7 mmol), methanol (9 V), and potassium carbonate (2.5 eq.) were placed, and the mixture was stirred at room temperature for one hour. After confirming the disappearance of the raw ingredient and the formation of the desired product by TLC (hexane/ethyl acetate = 1/3, the Rf value of the raw ingredient: 0.69, the Rf value of the desired product: 0.13), the mixture was concentrated to remove the methanol. The concentrate was diluted with water, and was extracted with ethyl acetate. The organic layer was washed with saturated saline, and was dried over sodium sulfate. After filtration and concentration, the solution was then purified by column chromatography (Biotage Sfar HC Duo, 350 g × 1, hexane/ethyl acetate = 1/1 to 0/1) to obtain 3-MMTr-thymidine in 35.0 g (at a yield of 79%).

### 1-4. 5'-MMTr Protection of 3-MMTr-thymidine

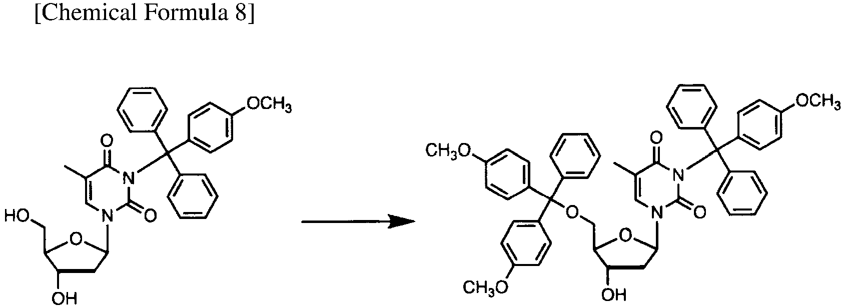

Into a 1 L single-neck round-bottom flask, the 3-MMTr-thymidine (67.0 mmol), pyridine (10 V), and MMTrCl (1.10 eq.) were placed, and the mixture was stirred at room temperature for 20 hours. After confirming the formation of the desired product by TLC (hexane/ethyl acetate = 1/3, the Rf value of the raw ingredient, the Rf value of the desired product, the mixture was concentrated to remove the pyridine. The concentrate was diluted with dichloromethane, washed with an aqueous solution of sodium bicarbonate, water, and saturated saline, and dried over sodium sulfate. After filtration and concentration, the solution was purified by column chromatography (Biotage Sfar HC Duo, 350 g × 3, hexane/ethyl acetate = 4/1 to 3/2) to obtain N3,5'-bis-MMTr-thymidine in 44.3 g (at a yield of 84%).

### 1-5. 3'-Amidite formation of N3,5'-bis-MMTr-thymidine

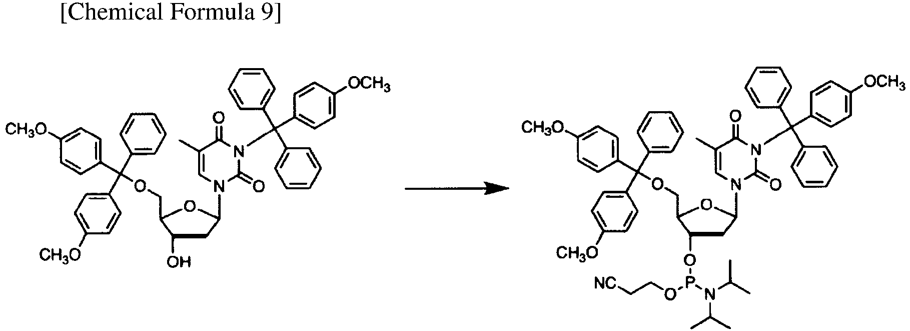

Into a 100 mL single-neck round-bottom flask, the N3,5'-bis-MMTr-thymidine (11.8 g, 15 mmol) was placed, and dichloromethane (75 mL) and 2-cyanoethyl bis(N,N'-diisopropyl)phosphoramidite (5.71 mL, 18 mmol) were added, and then the mixture was stirred at room temperature for 15 minutes. 1H-tetrazole (210 mg, 3 mmol) was added, and the mixture was stirred at room temperature for 15 minutes. An additional portion of *1H-*tetrazole (525 mg, 7.5 mmol) was then added, and the mixture was stirred at room temperature for 60 minutes. After confirming the disappearance of the raw ingredient and the formation of the desired product by TLC (hexane/ethyl acetate = 3/2), the reaction solution was directly subjected to silica gel column chromatography for purification (Biotage SnapUltra 500 g, hexane/ethyl acetate = 9/1 to 1/1) to obtain N3,5'-diacetylthymidine 3'-phosphoramidite in 8.65 g (at a yield of 58%).

### [Example 2] Bis-DMTr protection and 3'-amidite formation of 2'-deoxyadenosine

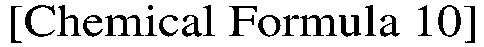

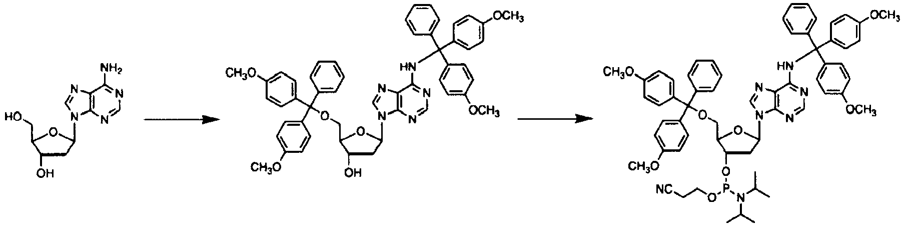

Into a 1 L single-neck round-bottom flask, 2'-deoxyadenosine (50.2 g, 200 mmol) was placed, and pyridine (10 V) and DMTrCl (1.10 eq.) were added. The mixture was stirred at room temperature for 20 hours. After confirming the formation of the desired product by TLC (hexane/ethyl acetate = 3/2, the Rf value of the raw ingredient: 0.0, the Rf value of the desired product: 0.11), the reaction solution was concentrated to remove the pyridine. The concentrate was diluted with dichloromethane, washed with an aqueous solution of sodium bicarbonate, water, and saturated saline, and dried over sodium sulfate. After filtration and concentration, the solution was purified by column chromatography (Biotage Sfar HC Duo, 350 g × 3, hexane/ethyl acetate = 4/1 to 3/2) to obtain N4,5'-bis-DMTr-2'-deoxyadenosine in 44.5 g (at a yield of 26%).

Into a 100 mL single-neck round-bottom flask, the N4,5'-bis-DMTr-2'-deoxyadenosine (12.8 g, 15 mmol) was placed, and dichloromethane (75 mL) and 2-cyanoethyl bis(N,N'-diisopropyl)phosphoramidite (5.71 mL, 18 mmol) were added. The mixture was stirred at room temperature for 15 minutes. 1H-tetrazole (210 mg, 3 mmol) was added, and the mixture was stirred at room temperature for 15 minutes. An additional portion of 1H-tetrazole (525 mg, 7.5 mmol) was then added, and the mixture was stirred at room temperature for 30 minutes. After confirming the disappearance of the raw ingredient and the formation of the desired product by TLC (hexane/ethyl acetate = 3/2), the reaction solution was directly subjected to silica gel column chromatography for purification (Biotage SnapUltra 500 g, hexane/ethyl acetate = 9/1 to 1/1) to obtain N4,5'-bis-DMTr-2'-deoxyadenosine 3'-phosphoramidite in 13.0 g (at a yield of 82%).

### [Example 3] Bis-DMTr protection and 3'-amidite formation of 2'-deoxycytidine

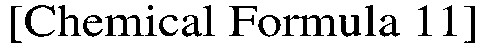

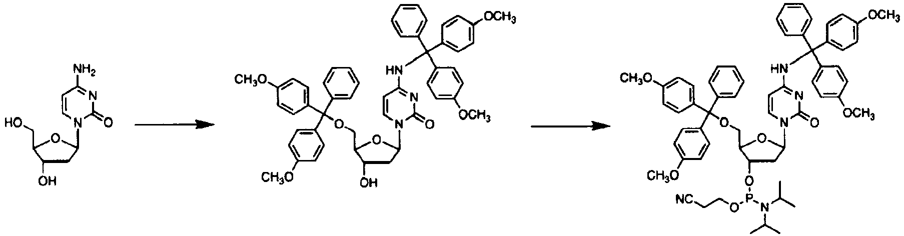

Into a 1 L single-neck round-bottom flask, 2'-deoxycytidine hydrochloride (52.5 g, 200 mmol) was placed, and pyridine (10 V) and DMTrCl (1.10 eq.) were added. The mixture was stirred at room temperature for 20 hours. After confirming the formation of the desired product by TLC (hexane/ethyl acetate = 2/3, the Rf value of the raw ingredient: 0.0, the Rf value of the desired product: 0.11), the reaction solution was concentrated to remove the pyridine. The concentrate was diluted with dichloromethane, washed with an aqueous solution of sodium bicarbonate, water, and saturated saline, and dried over sodium sulfate. After filtration and concentration, the solution was purified by column chromatography (Biotage Sfar HC Duo, 350 g × 3, hexane/ethyl acetate = 4/1 to 3/2) to obtain N4,5'-bis-DMTr-2'-deoxycytidine in 56.6 g (at a yield of 34%).

Into a 100 mL single-neck round-bottom flask, the N4,5'-bis-DMTr-2'-deoxycytidine (12.5 g, 15 mmol) was placed, and dichloromethane (75 mL) and 2-cyanoethyl bis(N,N'-diisopropyl)phosphoramidite (5.71 mL, 18 mmol) were added. The mixture was stirred at room temperature for 15 minutes. 1*H*-tetrazole (210 mg, 3 mmol) was added, and the mixture was stirred at room temperature for 15 minutes. An additional portion of 1*H*-tetrazole (525 mg, 7.5 mmol) was then added, and the mixture was stirred at room temperature for 30 minutes. After confirming the disappearance of the raw ingredient and the formation of the desired product by TLC (hexane/ethyl acetate = 3/2), the reaction solution was directly subjected to silica gel column chromatography for purification (Biotage SnapUltra 500 g, hexane/ethyl acetate = 9/1 to 1/1) to obtain N4,5'-bis-DMTr-2'-deoxycytidine 3'-phosphoramidite in 14.4 g (at a yield of 93%).

### [Example 4] Bis-DMTr protection and 3'-amidite formation of 2'-deoxyguanosine

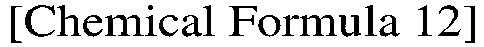

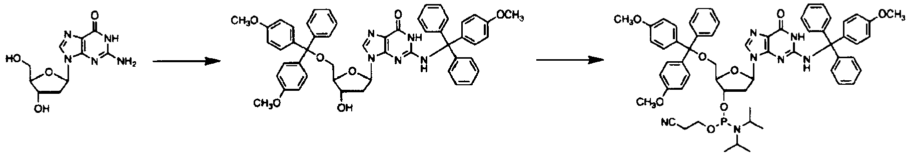

Into a 1 L single-neck round-bottom flask, 2'-deoxyguanosine hydrochloride (60.7 g, 200 mmol) was placed, and pyridine (10 V) and MMTrCl (1.10 eq.) were added. The mixture was stirred at room temperature for 20 hours. After confirming the formation of the desired product by TLC (hexane/ethyl acetate = 2/3, the Rf value of the raw ingredient: 0.0, the Rf value of the desired product: 0.11), the reaction solution was concentrated to remove the pyridine. The concentrate was diluted with dichloromethane, washed with an aqueous solution of sodium bicarbonate, water, and saturated saline, and dried over sodium sulfate. After filtration and concentration, the solution was purified by column chromatography (Biotage Sfar HC Duo, 350 g × 3, hexane/ethyl acetate = 1/1 to 1/9) to obtain N2,5'-bis-MMTr-2'-deoxyguanosine in 37 g (at a yield of 22%). The crude reaction product contained about 10% of Tris-DMTr derivative, which was removed at the purification stage.

Into a 100 mL single-neck round-bottom flask, the N2,5'-bis-MMTr-2'-deoxyguanosine (13.1 g, 15 mmol) was placed, and dichloromethane (75 mL) and 2-cyanoethyl bis(N,N'-diisopropyl)phosphoramidite (5.71 mL, 18 mmol) were added. The mixture was stirred at room temperature for 15 minutes. 1*H*-tetrazole (210 mg, 3 mmol) was added, and the mixture was stirred at room temperature for 15 minutes. An additional portion of 1*H*-tetrazole (525 mg, 7.5 mmol) was then added, and the mixture was stirred at room temperature for 360 minutes. After confirming the disappearance of the raw ingredient and the formation of the desired product by TLC (hexane/ethyl acetate = 3/2), the reaction solution was directly subjected to silica gel column chromatography for purification (Biotage SnapUltra 500 g, hexane/ethyl acetate = 1/1 to 1/4) to obtain N2,5'-bis-MMTr-2'-deoxyguanosine 3'-phosphoramidite in 13.6 g (at a yield of 85%).

### [Example 5] Oligonucleotide synthesis using bis-trityl amidite monomers

Using the apparatus and monomers below, and following the process and conditions described below, a 21-mer oligonucleotide was synthesized.

Synthesis apparatus: T-8, available from Nippon Techno Service Co., Ltd.
Solid support: PrimerSupport, available from GE Healthcare
Reagents other than monomers: reagents at the nucleic acid synthesizer grade, available from Glen Research
Washing solvent: Low-moisture acetonitrile, available from Wako Pure Chemical Industries

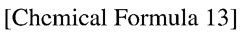

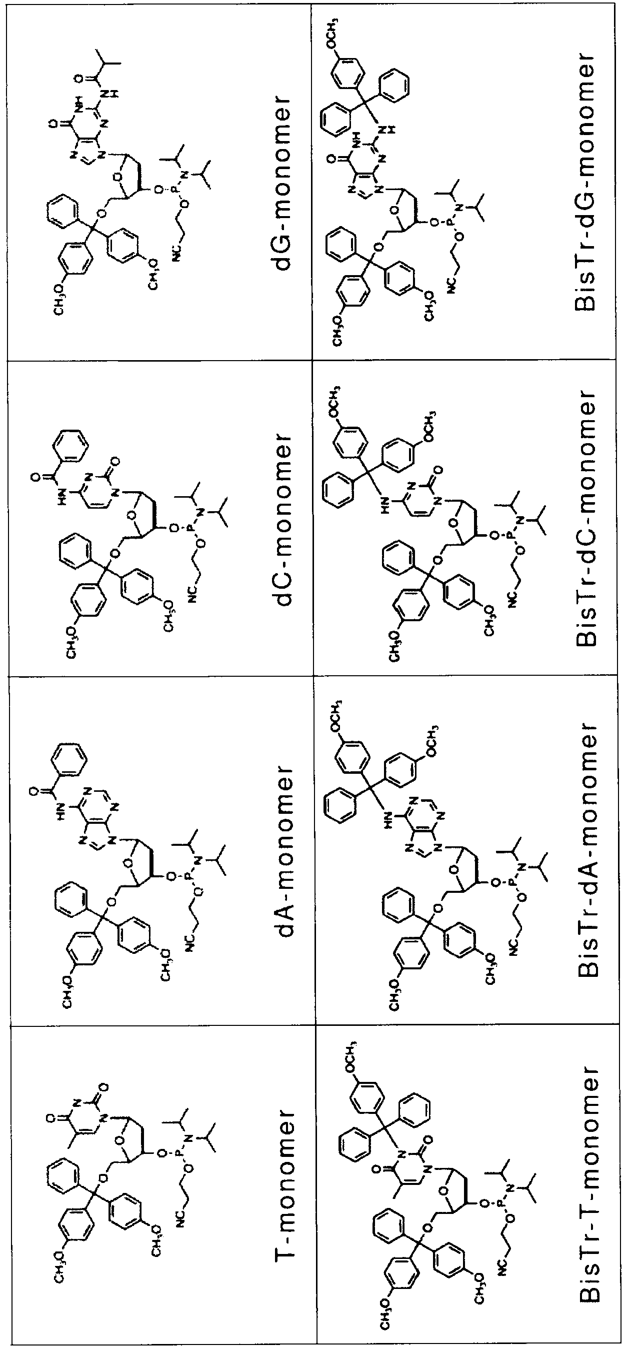

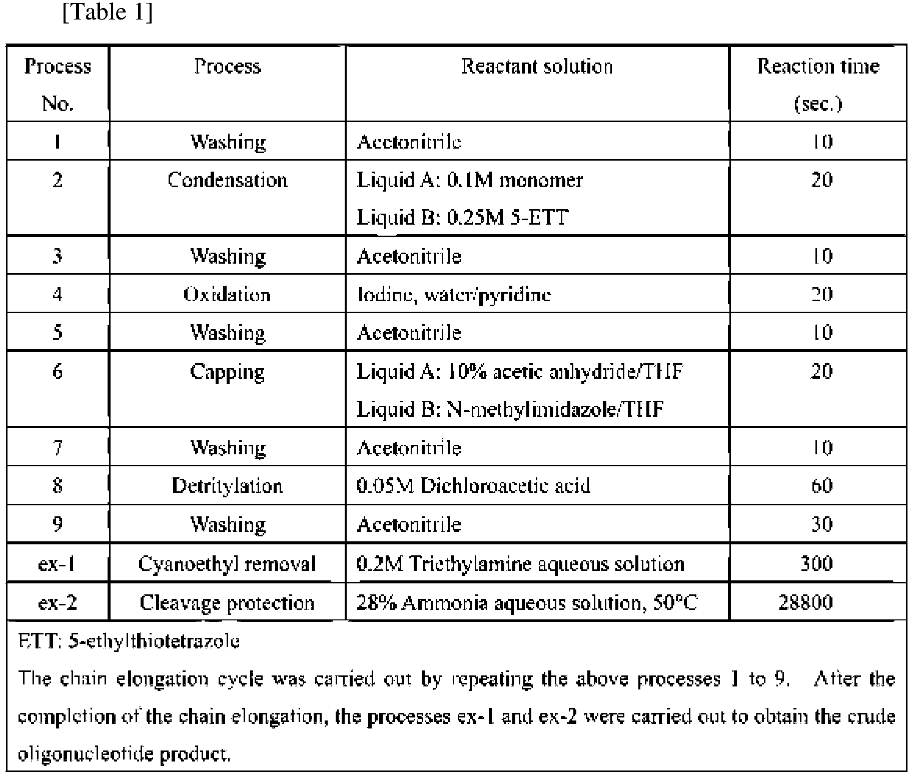

Purification conditions: Fractionation and purification Column: OASIS HLB + ion exchange → ODS
A: 0.1 M TEAA
B: 20% ACN / 0.1 M TEAA
   ↓
A: H₂O
B: 30% EtOH
   ↓
A: 10 mM NaOH
B: 10 mM NaOH / 2 M NaCl
Detritylation: 2% TFA, 100 mL

After treating the sample from the ion exchange column at 65°C for 12 hours, desalting was carried out with Glen Pak, and the sample was subjected to HPLC measurement.

The four synthesized sequences are shown in Table 2.

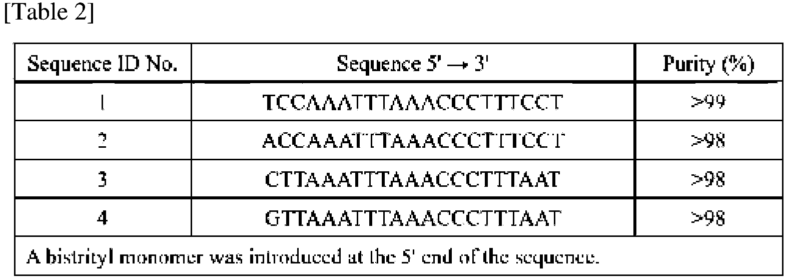

Fig. 1A and Fig. 1B illustrate the HPLC charts before and after the purification of the 21-mer having the sequence shown in the Sequence ID No. 1. Fig. 1A is the HPLC chart of the crude product that is 5'-bis-tritylated, and Fig. 1B is the HPLC chart after the purification of the product following the detritylation. From the obtained HPLC charts, it was confirmed that the purity of the 21-mer oligonucleotide having the desired sequence of the Sequence ID No. 1 was 99% or higher.

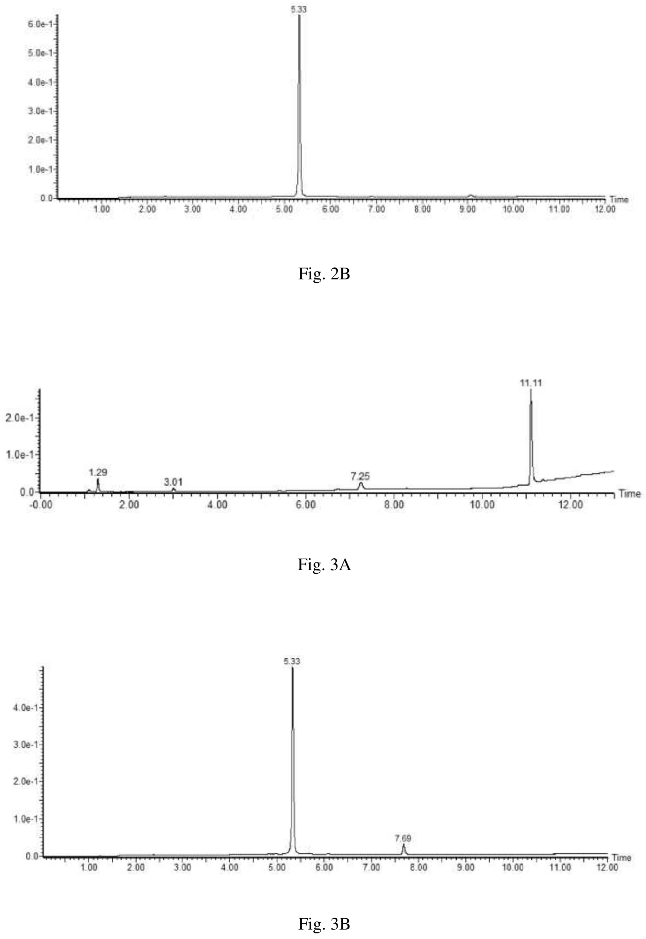

Fig. 2A and Fig. 2B illustrate the HPLC charts before and after the purification of the 21-mer having the sequence shown in the Sequence ID No. 4. Fig. 2A is the HPLC chart of the crude product that is 5'-bis-tritylated, and Fig. 2B is the HPLC chart after the purification of the product following detritylation. From the obtained HPLC charts, it was confirmed that the purity of the 21-mer oligonucleotide having the desired sequence of the Sequence ID No. 4 was 99% or higher.

### [Example 6] Tris-DMTr protection and 5'-amidite formation of guanosine

Into a 1 L single-neck round-bottom flask, guanosine hydrochloride (30.4 g, 100 mmol) was placed, and pyridine (10 V) and TBDMSC1 (0.95 eq.) were added. The mixture was stirred at room temperature for 15 minutes. After confirming the formation of the desired product by TLC (dichloromethane/methanol = 19/1), MMTrCl (4.0 eq.) was added, and the reaction solution was heated to 80°C and stirred for 20 hours. After cooling the reaction solution to room temperature, the pyridine was removed under a reduced pressure. The concentrate was diluted with dichloromethane, washed with an aqueous solution of sodium bicarbonate, water, and saturated saline, and dried over sodium sulfate. After filtration and concentration, the solution was purified by column chromatography (Biotage Sfar HC Duo, 350 g × 3, hexane/ethyl acetate = 4/1 to 1/4) to obtain 5'O-TBDMS-N2,2',3'-Tis-MMTr-guanosine in 45.9 g (at a yield of 36%).

To the 5'O-TBDMS-N2,2',3'-Tris-MMTr-guanosine (12.7 g, 10 mmol), a 0.1 M THF solution of TBAF (1.1 eq.) was added, and the mixture was stirred at room temperature for one hour. The reaction solution was dropped into pure water (1.0 L), and the precipitate was diluted with dichloromethane, washed with an aqueous solution of sodium bicarbonate, water, and saturated saline, and dried over sodium sulfate to obtain N2,2',3'-Tis-MMTr-guanosine.

Into a 100 mL single-neck round-bottom flask, the N2,2',3'-Tris-MMTr-guanosine (10.1 g, 10 mmol) was placed, and dichloromethane (75 mL) and 2-cyanoethyl bis(N,N'-diisopropyl)phosphoramidite (5.71 mL, 18 mmol) were added. The mixture was stirred at room temperature for 15 minutes. 1*H*-tetrazole (210 mg, 3 mmol) was added, and the mixture was stirred at room temperature for 15 minutes. An additional portion of 1*H*-tetrazole (525 mg, 7.5 mmol) was then added, and the mixture was stirred at room temperature for 360 minutes. After confirming the disappearance of the raw ingredient and the formation of the desired product by TLC (hexane/ethyl acetate = 3/2), the reaction solution was directly subjected to silica gel column chromatography for purification (Biotage SnapUltra 500 g, hexane/ethyl acetate = 1/1 to 1/4) to obtain N2,2',3'-Tris-MMTr-guanosine 5'-phosphoramidite in 11.6 g (at a yield of 85%).

## Claims

1. A highly lipophilic monomer building block for oligonucleotide synthesis, comprising a structure represented by the following Formula (I) or Formula (II): 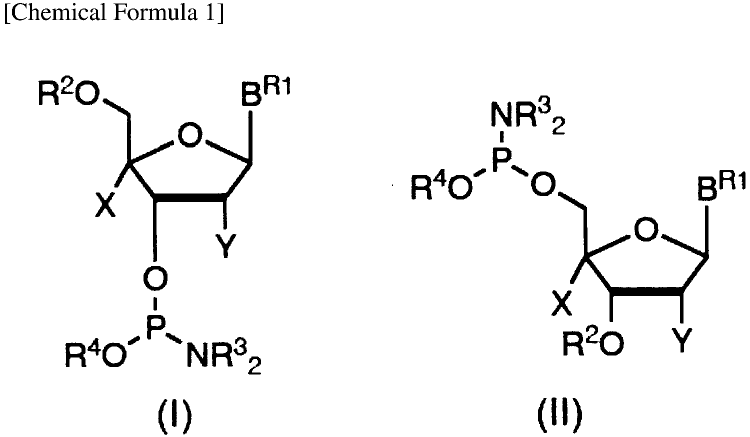 wherein, in the Formula (I) and Formula (II),
B is a naturally occurring or non-naturally occurring base;
R¹ is at least one of Tr, MMTr, DMTr, TMTr, TBDPS, TBS, Fmoc, benzoyl, isobutyryl, dmf, and Ac, provided that, in the Formula (II), when both OR² and Y are one of OMMTr, ODMTr, and OTMTr, R¹ is optionally free from a protecting group;
R² is one of Tr, MMTr, DMTr, TMTr, TBDPS, TBS, Fmoc, and 1-pyrenylmethyl;
R³ is CH₃, CH₂CH₃, or CH(CH₃)₂, or NR³₂ forms a ring that is -N(CH₂CH₂)₂ or - N(CH₂CH₂)₂O;
R⁴ is independently one of CH₂CH₂CN, CH₂CH=CH₂, CH₂(CF₂)₆CF₃, and CH₂(CF₂)₄CF₃;
X is H, or forms an X-Y bond together with Y; and
Y is one of F, OCH₃, OMOE, OTBDMS, and OR², or forms an X-Y bond, wherein X-Y is CH₂O, CH₂CH₂O, CH₂NH, CH₂NR', or CH₂N-N=NH₂, and R' is independently H, alkyl, carbamate, amide group, or substituted silyl.

2. The highly lipophilic monomer building block according to claim 1, wherein B^{R} is independently selected from A^{DMTr}, A^{TMTr}, A^{Ac}, C^{PMTr}, C^{TMTr}, C^{Ac}, G^{MMTr}, G^{Tr}, G^{ibu/TBDPS}, G^{dmf}, U^{Tr}, U^{TBDPS}, T^{MMTr}, and T^{TBDPS}.

3. A highly lipophilic nucleotide building block for oligonucleotide synthesis, comprising a structure represented by the following Formula (III) or the Formula (VI): 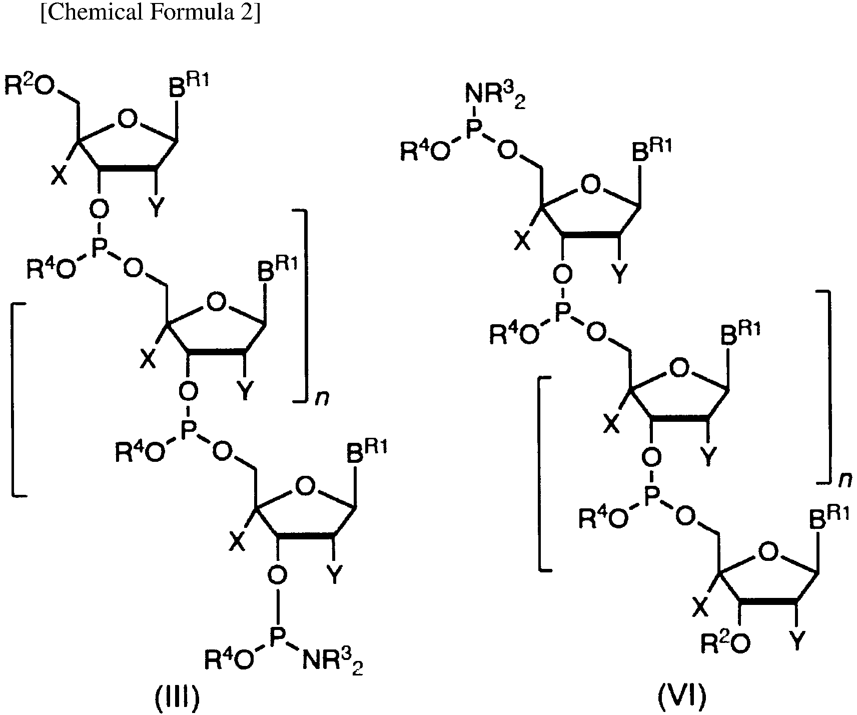 wherein, in the Formula (III) and Formula (VI),
B is a naturally occurring or non-naturally occurring base;
R¹ is at least one of Tr, MMTr, DMTr, TMTr, TBDPS, TBS, Fmoc, benzoyl, isobutyryl, dmf, and Ac, provided that, in the Formula (III) and Formula (VI), when both OR² and Y are one of OMMTr, ODMTr, and OTMTr, R¹ is optionally free from a protecting group;
R² is one of Tr, MMTr, DMTr, TMTr, TBDPS, TBS, Fmoc, and 1-pyrenylmethyl;
R³ is CH₃, CH₂CH₃, or CH(CH₃)₂, or NR³₂ forms a ring that is -N(CH₂CH₂)₂ or - N(CH₂CH₂O)₂;
R⁴ is independently one of CH₂CH₂CN, CH₂CH=CH₂, CH₂(CF₂)₆CF₃, and CH₂(CF₂)₄CF₃;
X is H, or forms an X-Y bond together with Y;
Y is one of F, OCH₃, OMOE, OTBDMS, and OR², or forms an X-Y bond, wherein X-Y is CH₂O, CH₂CH₂O, CH₂NH, CH₂NR', or CH₂N-N=NH₂, and R' is independently H, alkyl, carbamate, amide group, or substituted silyl; and
n is an integer from 0 to 46.

4. The highly lipophilic nucleotide building block according to claim 3, wherein B^{R} is independently selected from A^{DMTr}, A^{TMTr}, A^{Ac}, A, C^{DMTr}, C^{TMTr}, C^{Ac}, C, G^{DMTr}, G^{Tr}, G^{ibu/TBDPS}, G^{dmf}, G, U^{Tr}, U^{TBDPS}, U, T^{MMTr}, T^{BDPS}, and T.

5. The highly lipophilic nucleotide building block according to claim 3, wherein at least two of R¹, R2, and R4 are protected with Tr, MMTr, DMTr, TMTr, Fmoc, or TBDPS.

6. The highly lipophilic nucleotide building block according to claim 3, wherein n is an integer from 0 to 10.

7. A method of synthesizing an oligonucleotide using the highly lipophilic monomer building block according to claim 1 or the highly lipophilic nucleotide building block according to claim 3, comprising:
(a) a step of chain elongation in which an amidite portion of the highly lipophilic monomer building block according to claim 1 or the highly lipophilic nucleotide building block according to claim 3 is condensed with a hydroxyl group of a protected nucleoside or a protected nucleotide; and
(b) a step of deprotection of a protecting group of a hydroxyl group at one end of a chain-elongated product obtained in the chain elongation step,
wherein, the method comprises, after performing the step of chain elongation and the step of deprotection once or a predetermined number of times to obtain a protected oligonucleotide of a desired length,
(c) a step of deprotection of a phosphate protecting group, a protecting group of a nucleobase, and a protecting group of a hydroxyl group at a 3'- or 5'-end of the protected oligonucleotide to synthesize a protected oligonucleotide having a highly lipophilic protecting group at at least two positions;
(d) a step of separating the protected oligonucleotide having a highly lipophilic protecting group at at least two positions; and
(e) a step of deprotecting the separated protected oligonucleotide and purifying the obtained oligonucleotide.

8. The method of synthesizing an oligonucleotide according to claim 7, wherein the number of nucleosides contained in the oligonucleotide is in a range of 10 to 150.

9. The method of synthesizing an oligonucleotide according to claim 7, wherein the highly lipophilic protecting groups at at least two positions in the protected oligonucleotide obtained after the deprotection step (c) are Tr, MMTr, DMTr, TMTr, Fmoc, or TBDPS protecting groups.

10. The method of synthesizing an oligonucleotide according to claim 7, wherein the chain elongation step (a), the deprotection step (b), and the deprotection step (c) are carried out in a liquid phase.
